(19) Europäisches Patentamt / European Patent Office / Office européen des brevets

(11) **EP 4 600 345 A1**

(12) **EUROPEAN PATENT APPLICATION**
published in accordance with Art. 153(4) EPC

(43) Date of publication:
**13.08.2025 Bulletin 2025/33**

(21) Application number: **23874849.5**

(22) Date of filing: **03.10.2023**

(51) International Patent Classification (IPC):
*C12N 5/07* (2010.01)        *C08J 3/12* (2006.01)
*C12M 1/00* (2006.01)        *C12M 3/00* (2006.01)
*C12N 1/02* (2006.01)        *C12N 5/00* (2006.01)
*C12N 5/071* (2010.01)       *C12N 5/0735* (2010.01)
*C12N 5/074* (2010.01)       *C12N 5/0775* (2010.01)
*C12N 5/0789* (2010.01)      *C12N 5/0797* (2010.01)

(52) Cooperative Patent Classification (CPC):
**C08J 3/12; C12M 1/00; C12M 3/00; C12N 1/02; C12N 5/00; C12N 5/06**

(86) International application number:
**PCT/JP2023/036049**

(87) International publication number:
**WO 2024/075722 (11.04.2024 Gazette 2024/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.10.2022 JP 2022160144**
**04.10.2022 JP 2022160143**
**13.03.2023 JP 2023039008**
**29.05.2023 JP 2023087922**

(71) Applicant: **Tosoh Corporation**
**Yamaguchi 746-8501 (JP)**

(72) Inventors:
• **HIRATOKO, Shoya**
**Ayase-shi, Kanagawa 252-1123 (JP)**
• **NEZU, Yusuke**
**Ayase-shi, Kanagawa 252-1123 (JP)**
• **IMATOMI, Shinya**
**Ayase-shi, Kanagawa 252-1123 (JP)**
• **ITO, Hiroyuki**
**Ayase-shi, Kanagawa 252-1123 (JP)**

(74) Representative: **Novagraaf International SA**
**Chemin de l'Echo 3**
**1213 Onex, Geneva (CH)**

(54) **CELL CULTURE METHOD USING TEMPERATURE-RESPONSIVE MICROCARRIER, AND BEADS FOR TEMPERATURE-RESPONSIVE CELL CULTURE**

(57) The invention provides a method of culturing adherent cells in a culture vessel using microcarriers coated with a polymer that exhibits a lower critical solution temperature, the method comprising the following steps (1) to (4):
(1) a step of culturing the cells on the surfaces of the microcarriers in a culture solution at a temperature at or above the lower critical solution temperature,
(2) following step (1), a step of cooling the culture solution to a temperature at or below the lower critical solution temperature,
(3) following step (2), a step of stirring the culture solution in the culture vessel to detach the cells from the surfaces of the microcarriers, and
(4) following step (3), a step of recovering the cells detached from the surfaces of the microcarriers,
as well as temperature-responsive beads composed of a carrier and a polymer coat layer comprising a temperature-responsive polymer having a lower critical solution temperature of 0°C to 50°C, the carrier surface being positively charged and the thickness of the layer of the temperature-responsive polymer being 10 nm to 1000 nm.

**Description**

FIELD

The present invention relates to a cell culturing method using temperature-responsive microcarriers, and to temperature-responsive cell culturing beads and a method of culturing them.

BACKGROUND

With the ongoing development of biopharmaceuticals and regenerative medicine based on cellular sources, there are increasing demands for methods to culture cells more efficiently, in large-scale and high-quality. Among cells which are largely classified as suspension cells or adherent cells, the most useful cells are scaffold-dependent cells such as mesenchymal stem cells or pluripotent stem cells, which require a scaffold onto which the cells can adhere in order to proliferate. Although plastic dishes or flasks have been used in the prior art, according to the increase of demands for cells, culturing methods which employ microcarriers as suitable for mass culturing have become a greater focus of attention. In addition, microcarriers which are bead-like particles composed of synthetic polymers or natural polymers such as polysaccharides have also been developed. When microcarriers are loaded into a container with cell suspension and cultured, the surface area to which cells can adhere is expanded, whereby cell proliferation per unit volume of medium increases.

In culturing methods using microcarriers, cells and microcarriers in a culture solution are suspended by stirring so that the cells adhered on the surfaces of the microcarriers proliferate. Culturing methods using microcarriers when carried out under stirring conditions, can thus increase the cell density per unit volume compared with the conventional culturing methods using dishes or flasks.

However, it is also necessary to detach the proliferated adherent cells from the scaffold and recover them. Proteases such as trypsin are typically used for detachment and recovery of cells adhered to scaffolds. For recovery of cells proliferated on the surfaces of the microcarriers, in particular, it is common to use a protease such as trypsin for detachment from the microcarriers and subsequent recovery. However, the use of proteases such as trypsin might damage cells due to degradation of proteins on the cell surface, and a complicated procedure is also required for trypsin removal from recovered cells.

A cell recovery method that reduces the damaging of cells is disclosed in PTL 1, for example, as a method using microcarriers coated with a temperature-responsive polymer having a lower critical solution temperature. When the cells adhere and proliferate at a culturing temperature of 37°C, then are cooled to 20°C, to make the temperature-responsive polymer hydrophilized, the cells can be recovered without using a protease. However, such temperature-responsive microcarriers are in need of improvement because of their low cell-proliferative ability.

In PTL 2 and PTL 3, microcarriers coated with a polymer exhibiting a lower critical solution temperature are disclosed in order to solve the problems associated with detachment from microcarriers, when proteases are used in culturing methods using microcarriers. According to PTL 2 and PTL 3, the adhesive force on the surfaces of microcarriers is weakened by sol transition of a polymer that exhibits a lower critical solution temperature upon cooling, in order to detach the cells from the microcarriers and recover the cells. When recovering cells, it is difficult to completely detach the cells from the surfaces of the microcarriers by cooling alone, and therefore on the laboratory scale it is common to combine recovery with a pipetting procedure as described in PTL 3. However, because of the difficulties in applying pipetting procedures to mass culturing, the cell recovery methods described in PTL 2 and PTL 3 have not yet been applied in mass culturing techniques, and consequently cell culturing methods more suited for mass culturing are desired.

[CITATION LIST]

[PATENT LITERATURE]

[PTL 1] Japanese Patent No. 6313822
[PTL 2] Japanese Patent Publication No. 6954047
[PTL 3] Japanese Unexamined Patent Publication No. 2021-106543

SUMMARY

[TECHNICAL PROBLEM]

The first object of the present patent is to provide a cell recovery method carried out after cell culture with microcarriers coated with a polymer that exhibits a lower critical solution temperature, which allows the cell culturing method to be suited for mass culturing. The second object of the invention is to provide temperature-responsive microcarriers with highly cell-proliferative ability.

[SOLUTION TO PROBLEM]

In regard to the first object, the present inventors carried out much diligent research in light of the problems associated with culturing methods using microcarriers, and as a result completed this invention upon finding that if a cell culturing method comprising a cooling step and stirring step is carried out subsequent to cell culture with microcarriers coated with a polymer that exhibits a lower critical solution temperature, it is possible to recover the cells from the microcarriers at high efficiency and in a minimally invasive manner. Specifically, the present invention encompasses the following aspects.

<1-1>
A method for culturing adherent cells in a culture vessel using microcarriers coated with a polymer that exhibits a lower critical solution temperature, the method comprising the following steps (1) to (4):

(1) a step of culturing the cells on the surfaces of the microcarriers in a culture solution at a temperature at or above the lower critical solution temperature,
(2) following step (1), a step of cooling the culture solution to a temperature at or below the lower critical solution temperature,
(3) following step (2), a step of stirring the culture solution in the culture vessel to detach the cells from the surfaces of the microcarriers, and
(4) following step (3), a step of recovering the cells detached from the surfaces of the microcarriers.

<1-2>
The method according to <1-1> above, wherein in step (3), stirring is carried out with a stirring Reynolds number in the range of 50 to 2000.
<1-3>
The method according to <1-1> or <1-2>, wherein step (2) is carried out by exchanging 10 (v/v)% to 90 (v/v)% of the culture solution used in step (1) with culture solution cooled to a temperature at or below the lower critical solution temperature.
<1-4>
The method according to any one of <1-1> to <1-3> above, wherein step (4) is carried out by removing the microcarriers using a mesh.
<1-5>
The method according to any one of <1-1> to <1-4>, wherein the particle diameters of the microcarriers are 50 $\mu$m to 1000 $\mu$m.
<1-6>
The method according to any one of <1-1> to <1-5>, wherein the lower critical solution temperature is 0°C to 50°C.
<1-7>
The method according to any one of <1-1> to <1-6> above, wherein the adherent cells are stem cells.

In regard to the second object, the present inventors carried out much diligent research in light of the problem of low proliferation of cells with temperature-responsive microcarriers, and as a result completed this invention upon finding that beads obtained by coating positively charged beads with a temperature-responsive polymer having a lower critical solution temperature of 0°C to 50°C are able to be satisfactorily adhered by cells, and that the cells can be detached and recovered by cooling after culturing is completed. Specifically, the present invention further encompasses the following aspects.

<2-1>
Temperature-responsive beads comprising a carrier and a polymer coat layer made of a temperature-responsive polymer having a lower critical solution temperature of 0°C to 50°C, wherein the carrier surface is positively charged, and the thickness of the layer of the temperature-responsive polymer is 10 nm to 1000 nm.
<2-2>

Temperature-responsive beads according to <2-1> above, wherein the positively charged component of the carrier surface includes a tertiary amine, a quaternary ammonium salt or an alkaline earth metal salt.
<2-3>
The beads according to <2-1> or <2-2> above, wherein the specific gravity of the carrier is 1.0 to 1.1.
<2-4>
The temperature -responsive beads according to any one of <2-1> to <2-3>, wherein the material of the carrier is polystyrene.
<2-5>
The temperature-responsive beads according to any one of <2-1> to <2-4>, wherein the particle diameter of the carrier is 50 $\mu$m to 1000 $\mu$m.
<2-6>
A cell culturing method using temperature-responsive beads according to any one of <2-1> to <2-5> above.

[ADVANTAGEOUS EFFECTS OF INVENTION]

For a cell culturing method with microcarriers coated with a polymer that exhibits a lower critical solution temperature, a method comprising a cooling step and a stirring step allows cells to be recovered from the microcarriers at high efficiency and in a minimally invasive manner.

Moreover, temperature-responsive beads comprising a carrier and a polymer coat layer made of a temperature-responsive polymer having a lower critical solution temperature of 0°C to 50°C, wherein the carrier surface is positively charged, have highly cell-proliferative ability and allow the cells to be recovered by further cooling.

DESCRIPTION OF EMBODIMENTS

Embodiments for carrying out the invention will now be described, but they are not intended to limit the gist of the invention. The present invention may be implemented with appropriate modifications that are within the scope of its gist.

<Method of culturing adherent cells in culture vessel using microcarriers coated with a polymer that exhibits a lower critical solution temperature>

According to one aspect, the invention provides a cell culturing method using temperature-responsive microcarriers. The present invention is a method of culturing adherent cells in a culture vessel using microcarriers coated with a polymer that exhibits a lower critical solution temperature, the method comprising the following steps (1) to (4).

(1) a step of culturing the cells on the surfaces of the microcarriers in a culture solution at a temperature at or above the lower critical solution temperature,
(2) following step (1), a step of cooling the culture solution to a temperature at or below the lower critical solution temperature,
(3) following step (2), a step of stirring the culture solution in the culture vessel to detach the cells from the surfaces of the microcarriers, and
(4) following step (3), a step of recovering the cells detached from the surfaces of the microcarriers.

The lower critical solution temperature (LCST) is the phase separation temperature, lower than which the polymer dissolves in water producing a transparent solution, and higher than which it is insolubilized in water, producing opaqueness or a precipitate. The LCST is not particularly limited but is preferably near the culturing temperature, and as an example it may be in the range 0°C to 50°C, preferably 20°C to 40°C and even more preferably 25°C to 35°C.

Homopolymer repeating units exhibiting a lower critical solution temperature, and their lower critical solution temperatures for water are, for example: N-isopropylacrylamide (LCST = 32°C), N-n-propylmethacrylamide (LCST = 22°C), N-tetrahydrofurfurylacrylamide (LCST = 28°C), N-ethoxyethyl acrylamide (LCST = 35°C), N,N-diethylacrylamide (LCST = 32°C), N-*n*-propylmethacrylamide (LCST = 28°C), N-tetrahydrofurfurylmethacrylamide (LCST = 35°C), N-methyl-N-isopropylacrylamide (LCST = 23°C) and N-methyl-N-n-propylacrylamide (LCST = 20°C). The lower critical solution temperature that is exhibited by a given concentration of an aqueous solution is approximate, but N-isopropylacrylamide is preferred for its lower concentration-dependency of the lower critical solution temperature.

The repeating unit of the polymer that exhibits lower critical temperature used for the invention may be a single type or a combination of two or more types. So long as the polymer exhibits an LCST, it may also include a repeating unit of a polymer that does not exhibit an LCST, in addition to the repeating unit of the polymer that does exhibit an LCST. Another polymer compound may also be included in addition to the polymer that exhibits an LCST, depending on the purpose, such as improving adhesiveness of the cells or microcarriers. In the Examples of the present invention, for example, a polymer compound comprising carboxystyrene and styrene is introduced to improve the cell adhesion. As an example, the composition of N-isopropylacrylamide, a polymer that exhibits an LCST, is 10 mol% to 95 mol%, preferably 30 mol% to 80 mol% and even more preferably 65 mol% to 70 mol%.

The material of the microcarriers is not particularly restricted and may be polystyrene, polymethyl methacrylate, polyethylene terephthalate, polycarbonate, cellulose, cyclodextrin, acrylamide, an alginic acid salt, dextran, gelatin or glass, or a mixture of two or more of the same, for example.

The shapes of the microcarriers are also not particularly restricted and may be spherical, oval spherical, flat or tubular, for example. The microcarriers may be either porous or non-porous. When porous, there is no restriction on the pore size.

The diameters of the microcarriers are not particularly restricted, but the long diameters of the microcarriers are preferably 50 $\mu$m to 1000 $\mu$m and more preferably 100 to 700 $\mu$m. If the lengths are smaller than this range, separation from the cells becomes more difficult and the recovery rate is lower. Long diameters larger than this range reduce the culturing area per volume.

As used herein, the term "temperature-responsive microcarriers" refers to microcarriers coated with a polymer that exhibits a lower critical solution temperature.

The method of coating the polymer that exhibits lower critical temperature onto the microcarriers is not particularly restricted, and for example, it may be a method of chemically coating a repeating unit that exhibits a lower critical solution temperature using electron beam irradiation, or a method of physically covering the microcarriers by coating with a surface treatment agent comprising the polymer that exhibits a lower critical temperature dissolved in a solvent.

The surfaces of the microcarriers coated with the polymer exhibiting an LCST may be further coated with an extracellular matrix. The type of extracellular matrix is not particularly restricted and may be a Matrigel composed mainly of collagen, atelocollagen, hyaluronic acid, elastin, proteoglycan, glycosaminoglycan, fibronectin, laminin, vitronectin, gelatin or laminin, collagen IV, heparan sulfate proteoglycan or entactin/nidogen-1,2, for example, either as a single type or a combination of two or more types. Segments of such extracellular matrix components may also be used.

Adherent cells are cells that adhere to the surface of a cell culture support, such as microcarriers. The origin of cells of the cells is not particularly restricted, and examples include a human, monkey, dog, cat, rabbit, rat, nude mouse, mouse, guinea pig, pig, sheep, Chinese hamster or cow. Examples of specific cells include various cultured cell lines such as Chinese hamster ovary-derived CHO cells, African green monkey kidney-derived Vero cells, mouse connective tissue L929 cells, human embryonic kidney-derived HEK293 cells and human cervical cancer-derived HeLa cells, epithelial cells and endothelial cells making up different tissues and organs in the body, skeletal muscle cells, smooth muscle cells, myocardial cells which exhibit contractive properties, neuron cells of the nervous system, glial cells, fibroblasts, organism immunity-related macrophages and dendritic cells, organism metabolism-related liver parenchymal cells, liver non-parenchymal cells and adipocytes, cells with differentiation potency, including various types of stem cells such as induced pluripotent stem (iPS) cells, embryonic stem (ES) cells, embryonic germline (EG) cells, embryonic cancer (EC) cells, mesenchymal stem cells, hepatic stem cells, pancreatic stem cells, skin stem cells, muscle stem cells and germ stem cells, or progenitor cells of different tissues, as well as cells that have differentiated from such cells, among which mesenchymal stem cells are especially preferred for use. Mesenchymal stem cells are stem cells that are able to differentiate into all or some mesenchymal cells such as chondrocytes, osteoblasts and adipocytes, as well as their progenitor cell groups. The source of the mesenchymal stem cells is not particularly restricted and may be derived from tissues such as bone marrow, fat, dental pulp, umbilical cord blood, placenta or synovial membrane, or pluripotent stem cells such as ES cells or iPS cells.

In step (1), the polymer that exhibits a lower critical solution temperature is gelled when the temperature of the culture solution in the culture vessel is a temperature at or above the lower critical solution temperature, thus allowing the adherent cells to adhere to and proliferate on the microcarriers. When using human-derived cells it is recommended to carry out the step at a temperature close to body temperature to obtain high culturing efficiency, a preferred temperature range being 30°C to 40°C and a more preferred temperature range being 36°C to 38°C. There are no particular restrictions on the conditions other than the lower critical solution temperature, and for example, stationary culture or suspension culture may be carried out, although suspension culture is preferred to increase the culture area per unit volume.

The cell culture density is not particularly restricted so long as the cells adhere and proliferate, but for human-derived mesenchymal stem cells it is, for example, preferably $1.0 \times 10^1$ cells/cm$^2$ to $1.0 \times 10^5$ cells/cm$^2$ and more preferably $1.0 \times 10^2$ cells/cm$^2$ to $1.0 \times 10^4$ cells/cm$^2$, per surface area of the microcarriers. The other culturing conditions are not particularly restricted, and the culturing may be carried out under conditions commonly used in the relevant field.

The temperature of the culture solution during cooling in step (2) is preferably a temperature of at least 1°C lower than the lower critical solution temperature. The method of lowering the temperature of the microcarriers (cooling treatment) may be exchanging the liquid in the culture vessel with a cooled liquid, or cold storage, but exchange with a cooled liquid is preferred in order to shorten the time required for cooling. The amount of exchange with the cooled liquid is not particularly restricted, but it is preferably 10% to 90% as an example, and more preferably 50% to 90% in order to increase the cooling rate. The cooled liquid is not particularly restricted and may be selected as a culture solution or other culture medium solution or isotonic solution, as appropriate for the purpose. The cooling time is preferably 5 to 60 minutes.

The stirring method in step (3) is not particularly restricted, and may be stirring of the culture solution by center stirring in which a vertically installed stirring blade at the center of the culture vessel is rotated. The degree of stirring is not particularly restricted but is preferably stirring with a stirring Reynolds number (indicating the degree of stirring) in a range of 50 to 2000, more preferably in a range of 100 to 1800 and even more preferably in a range of 300 to 1500. If the stirring Reynolds number is less than 50, the cells will fail to completely detach from the microcarriers, thus lowering the recovery rate. If the stirring Reynolds number is greater than 2000, the cells may be damaged, resulting in a lower viable cell rate. The stirring Reynolds number is represented by the following formula (1).

$$\text{Stirring Reynolds number} = \text{Fluid density} \times \text{stirring speed} \times \text{stirring diameter/fluid viscosity}$$

$$\text{Formula (1)}$$

The temperature of the liquid in the culture vessel in step (3) is preferably at least 1°C lower than the lower critical solution temperature in order to prevent the detached cells from again adhering to the microcarriers. The liquid during stirring is not particularly restricted and may be selected to be a culture solution or other culture medium solution or isotonic solution, as appropriate for the purpose. The stirring time is preferably 1 to 60 minutes in consideration of the cell recovery rate and of avoiding damage.

The method for separating the microcarriers from the cells in step (4) is not particularly restricted and may be a method of separation based on differences in sedimentation rate, or a method of separation based on differences in particle diameter. The method of separation based on particle diameter may be a method of separating the microcarriers from the cell suspension using a mesh, for example. The mesh material is not particularly restricted, and nylon is an example. The mesh opening is not particularly restricted and may be 20 $\mu$m to 100 $\mu$m, preferably 30 $\mu$m to 80 $\mu$m and even more preferably 40 $\mu$m to 60 $\mu$m, for example. A small mesh opening prevents the cells from passing through the mesh, thus lowering the cell recovery rate. A large mesh opening makes it difficult to achieve separation since the microcarriers also pass through the mesh.

The cell culturing vessel of the invention is not particularly restricted so long as it is a vessel allowing stirring and suspension of the microcarriers. In the Examples of the invention, a 30 mL single use bioreactor (Product No. BWV-S03A by Able Corp.) is used.

The composition of the culture solution used in culturing according to the invention is not particularly restricted so long as the cells adhere and proliferate, and it may contain basic culture solution and serum, as well as antibiotics. The type of basic culture solution is also not particularly restricted and may be MEM, $\alpha$MEM, DMEM, EMEM, GMEM, DMEM/Ham's F-12, Ham's F-12, Ham's F-10, Medium 199 or RPMI 1640, for example. The type of serum is not particularly restricted and may be fetal bovine serum (FBS), calf serum, adult bovine serum, horse serum, sheep serum, goat serum, pig serum, chicken serum, rabbit serum or human serum, with FBS being most commonly used due to its availability. The serum concentration in the culture solution is not particularly restricted. While commonly used at a concentration of 20 vol% or lower for cost effectiveness, the concentration may also be higher than 20 vol%. The medium may also be serum-free medium lacking untreated or unpurified serum, and comprising purified blood-derived components or animal tissue-derived components (growth factors).

The method of measuring the viable cell rate is not particularly restricted, and a known example is a method of assessing cell viability based on Trypan blue staining, whereby the cytoplasm of dead cells is stained blue. The method of assessing cell viability and determining the cell count is not particularly restricted, and may be a method of manual assessment by the operator using a hemocytometer, or a method of automatic assessment by an automatic cell counter, with the latter method using an automatic cell counter being preferred to allow counting without being dependent on operator proficiency.

<Temperature-responsive cell culturing beads and culturing method>

According to a second aspect, the invention provides temperature-responsive cell culturing beads and a culturing method. The temperature-responsive beads of the invention comprise positively charged beads as the carrier, coated with a temperature-responsive polymer having a lower critical solution temperature of 0°C to 50°C.

The construction of the positively charged beads as the carrier of the invention is not particularly restricted. As an example, a positively charged compound may be chemically anchored, or physically anchored, onto the carrier surfaces. The method of chemical anchoring may be, for example, a method of copolymerization of a positively charged monomer during production of the carrier, or a method of chemical modification with positively charged functional groups on particle surfaces. The functional groups to be positively charged are not particularly restricted, and examples include tertiary amino groups, quaternary ammonium groups and alkaline earth metal salts, with trimethylammonium, dimethylamino, diethylamino and calcium phosphate being particularly preferred for their ease of modification. Examples of methods of anchoring by physical adsorption include a method of covering the carrier with a cationic polymer, and a method of covering the surfaces with an inorganic compound having low solubility in water.

The construction of the positively charged beads as the carrier is not particularly restricted, but it preferably has a specific gravity of 1.0 to 1.1 and more preferably 1.01 to 1.06, as this will allow more gentle precipitation in the culture medium. A specific gravity of lower than 1.0 will tend to result in floating in the culture medium, thus interfering with culturing, while a value of higher than 1.1 will tend to interfere with dispersion in the culture medium. The material of the carrier is not particularly restricted, and examples include synthetic polymers, including resins of polyethylene, polypropylene, polystyrene, polyalkyl (meth)acrylate, polyalkyl (meth)acrylamide, polyester, polyurethane, polyvinyl chloride and polycarbonate, as well as their copolymers, plant-derived polymers such as dextran and cellulose, and wood chips or ceramics. The specific gravity of the beads can be designed to approximate the specific gravity of the culture solution, and from the viewpoint of inhibiting sedimentation of the beads during stirred cell culturing, the carrier is preferably a synthetic polymer bead carrier of polystyrene, polyalkyl (meth)acrylate, polyalkyl (meth)acrylamide, polyester or polyurethane, and more preferably a polystyrene bead carrier. The material forming the bead carrier is also preferably crosslinked in order to inhibit elution into the culture medium. The shape of the bead carrier is not particularly restricted, and it may be laminar or spherical, or provided as porous bodies. When the bead carrier is spherical, the particle diameters are not particularly restricted but are preferably 50 μm to 1000 μm, more preferably 150 μm to 600 μm and even more preferably 150 μm to 500 μm, since large particle diameters interfere with dispersion in the culture solution and small diameters interfere with cell adhesion.

As used herein, the "lower critical solution temperature" (LCST) is the temperature at which solubility in water changes for certain types of polymers, and specifically, polymers having an LCST undergo dehydration on the high-temperature end from the LCST, exhibiting stronger hydrophobic interaction, while undergoing hydration at the lower temperature end from the LCST, resulting in swelling or dissolution.

A "temperature-responsive polymer", for the purpose of the invention, is a polymer containing a repeating unit of a monomer whose homopolymer has an LCST of 0°C to 50°C for water.

The structure of the temperature-responsive polymer of the invention may include a repeating unit which exhibits an LCST and a repeating unit which does not exhibit an LCST, as a separate structure. A repeating unit with an LCST is preferably in a block structure, in order to exhibit a satisfactory temperature-responsive property. When temperature-responsive beads of the invention are used for cell culturing, the LCST of the repeating unit with an LCST is preferably 0°C to 50°C, more preferably 10°C to 40°C and even more preferably 20°C to 35°C, for use in general culturing near 37°C. If the LCST is below 0°C or higher than 50°C, the cells may suffer damage, making it difficult to accomplish minimally invasive cell detachment. Examples of repeating units having LCST of 0°C to 50°C include repeating units produced by polymerization of the monomers N-isopropylmethacrylamide (LCST = ~44°C), N-ethoxyethyl acrylamide (LCST = ~35°C), N-tetrahydrofurfurylmethacrylamide (LCST = ~35°C), N-isopropylacrylamide (LCST = ~32°C), N,N-diethylacrylamide (LCST = ~32°C), N-*n*-propylmethacrylamide (LCST = ~28°C), N-tetrahydrofurfurylacrylamide (LCST = ~28°C), N-methyl-N-isopropylacrylamide (LCST = ~22°C), N-*n*-propylacrylamide (LCST = ~22°C) and N-methyl-N-*n*-propylacrylamide (LCST = ~20°C). The temperature-responsive polymer is not particularly restricted, and examples include (N-isopropylacrylamide)-(*n*-butyl methacrylate) block copolymer and (N-isopropylacrylamide)-(*n*-butyl acrylate) block copolymer described in Japanese Patent No. 5846584, (2-dimethylaminoethyl methacrylate)-(*n*-butyl methacrylate)-(N-isopropylacrylamide) block copolymer described in Japanese Patent No. 6954047, and (N-isopropylacrylamide)-(*n*-butyl methacrylate)-(2-methoxyethyl acrylate) block copolymer described in Japanese Patent No. 7127330.

The thickness of the layer of the temperature-responsive polymer of the temperature-responsive beads is not particularly restricted but is preferably 10 nm to 1000 nm and more preferably 50 nm to 500 nm. A thickness of smaller than 10 nm reinforces the positive charge effect, leading to poor temperature-responsiveness, while a thickness of larger than 1000 nm weakens the positive charge effect.

The method for anchoring the temperature-responsive polymer coat layer onto the carrier is not particularly restricted, and may be chemical anchoring or physical adsorption of the polymer. The method used for anchoring by physical adsorption is not particularly restricted and may be a method of spraying the polymer solution onto the bead carrier followed by drying, for example. The drying method is also not particularly restricted and may be air-drying or drying under reduced pressure. When the carrier is porous, it may be immersed in a solvent for several hours for degassing prior to coating the temperature-responsive polymer.

The temperature-responsive beads of the invention may be used as microcarriers for cell culturing. The type of cells used is not particularly restricted so long as they adhere to the temperature-responsive beads before the cooling for detachment of the cells, and they may be derived from a human, monkey, dog, cat, rabbit, rat, nude mouse, mouse, guinea pig, pig, sheep, Chinese hamster or cow, for example. Examples of specific cells include various cultured cell lines such as Chinese hamster ovary-derived CHO cells, African green monkey kidney-derived Vero cells, mouse connective tissue L929 cells, human embryonic kidney-derived HEK293 cells and human cervical cancer-derived HeLa cells, epithelial cells and endothelial cells making up different tissues and organs in the body, skeletal muscle cells, smooth muscle cells, myocardial cells which exhibit contractive properties, neuron cells of the nervous system, glial cells, fibroblasts, organism immunity-related macrophages and dendritic cells, organism metabolism-related liver parenchymal cells, liver non-parenchymal cells and adipocytes, cells with differentiation potency, including various types of stem cells such as induced pluripotent stem (iPS) cells, embryonic stem (ES) cells, embryonic germline (EG) cells, embryonic cancer (EC) cells, mesenchymal stem cells, hepatic stem cells, pancreatic stem cells, skin stem cells, muscle stem cells and germ stem cells, or progenitor cells of different tissues, as well as cells that have differentiated from such cells, among which mesenchymal stem cells are especially preferred for use. Mesenchymal stem cells are stem cells that are able to differentiate into all or some mesenchymal cells such as chondrocytes, osteoblasts and adipocytes, as well as their progenitor cell groups. The source of the mesenchymal stem cells is not particularly restricted and may be derived from tissues such as bone marrow, fat, dental pulp, umbilical cord blood, placenta or synovial membrane, or pluripotent stem cells such as ES cells or iPS cells.

The composition of the culture solution used for cell culturing using temperature-responsive beads of the invention is not particularly restricted so long as the cells adhere and proliferate, and it may contain basic culture solution and serum, as well as antibiotics. The type of basic culture solution is also not particularly restricted and may be MEM, $\alpha$MEM, DMEM, EMEM, GMEM, DMEM/Ham's F-12, Ham's F-12, Ham's F-10, Medium 199 or RPMI 1640, for example. The type of serum is not particularly restricted and may be fetal bovine serum (FBS), calf serum, adult bovine serum, horse serum, sheep serum, goat serum, pig serum, chicken serum, rabbit serum or human serum, with FBS being most commonly used due to its availability. The serum concentration in the culture solution is not particularly restricted. While commonly used at a concentration of 20 vol% or lower for cost effectiveness, the concentration may also be higher than 20 vol%. The medium may also be serum-free medium lacking untreated or unpurified serum, and comprising purified blood-derived components or animal tissue-derived components (growth factors).

In cell culturing using temperature-responsive beads of the invention, the cells are cultured on the temperature-responsive beads at a higher temperature than the LCST of the temperature-responsive polymer, and after cell proliferation, the proliferated cells are detached from the beads at a temperature at or below the LCST of the temperature-responsive polymer. The cell culturing method is not particularly restricted, and for example, culturing of the cells may be carried out by adding temperature-responsive beads of the invention to a cell culturing vessel containing culture medium and seeding the cells, and then allowing them to stand, continuously stirring, or stirring or shaking for a predetermined period of time. The cooling method used for detachment of the cells from the temperature-responsive beads of the invention by cooling is not particularly restricted, and it may be cooling in a cold area or exchange of the culture medium with cooled medium. For efficient detachment of the cells, the culture support may be lightly tapped or shaken, or the culture solution may be stirred, or pipetting may also be carried out.

The seeding density is not particularly restricted so long as the cells adhere and proliferate, but for human-derived mesenchymal stem cells it is, for example, preferably $1.0 \times 10^1$ cells/cm$^2$ to $1.0 \times 10^5$ cells/cm$^2$ and more preferably $1.0 \times 10^2$ cells/cm$^2$ to $1.0 \times 10^4$ cells/cm$^2$ per surface area of the microcarriers. The other culturing conditions are also not particularly restricted, and the culturing may be carried out under conditions commonly used in the relevant field.

EXAMPLES

Examples of the invention will now be described, with the understanding that the invention is not to be limited in any way by the Examples. Unless otherwise specified, the reagents used were commercial products.

<Method of culturing adherent cells in culture vessel using microcarriers coated with polymer exhibiting lower critical solution temperature>

Reference Example 1: Synthesis of temperature-responsive polymer 1

After adding 0.650 g (5 mmol) of 2-methoxyethyl acrylate (MEA) to a 200 mL 2-necked flask, 31.8 mg (100 $\mu$mol) of cyanomethyldodecyl trithiocarbonate, 1.6 mg (10 $\mu$mol) of azobisisobutyronitrile and 10 mL of *tert*-butyl alcohol were added, and argon gas exchange was performed, after which the mixture was heated and stirred for 24 hours at 62°C.
After the first heating and stirring, 3.845 g (30 mmol) of *n*-butyl acrylate (BA) was added, 1.6 mg (10 $\mu$mol) of azobisisobutyronitrile and 5 mL of *tert*-butyl alcohol were further added, and argon gas exchange was performed, after which the mixture was heated and stirred for 24 hours at 62°C.
After the second heating and stirring, 7.355 g (65 mmol) of N-isopropylacrylamide (IPAAm LCST = 32°C) was added, 1.6 mg (10 $\mu$mol) of azobisisobutyronitrile and 85 mL of *tert*-butyl alcohol were further added, and argon gas exchange was performed, after which the mixture was heated and stirred for 24 hours at 62°C.
After the third heating and stirring, the reaction mixture was purified by reprecipitating purification with water and dried under reduced pressure to obtain a yellow solid. The obtained yellow solid was dissolved in chloroform and the chloroform phase was recovered using a separatory funnel. The recovered chloroform phase was concentrated with an evaporator and purified by reprecipitating purification in heptane. The precipitate was collected by filtration and dried under reduced pressure to obtain 8.295 g of temperature-responsive polymer 1, poly (MEA-BA-IPAAm). The composition of the obtained temperature-responsive polymer 1 was MEA:BA:IPAAm = 5:30:65 (mol%), the lower critical solution temperature was 32°C, the Mn was $11.8 \times 10^4$, and the Mw/Mn ratio was 1.45.

Reference Example 2: Synthesis of temperature-responsive polymer 2

After adding 3.845 g (30 mmol) of n-butyl acrylate (BA) to a 200 mL 2-necked flask, 31.8 mg (100 μmol) of cyanomethyldodecyl trithiocarbonate, 1.6 mg (10 μmol) of azobisisobutyronitrile and 15 mL of *tert*-butyl alcohol were added, and argon gas exchange was performed, after which the mixture was heated and stirred for 24 hours at 62°C.

After the heating and stirring, 7.355 g (65 mmol) of N-isopropylacrylamide (IPAAm LCST = 32°C) was added, 1.6 mg (10 μmol) of azobisisobutyronitrile and 85 mL of *tert*-butyl alcohol were further added, and argon gas exchange was performed, after which the mixture was further heated and stirred for 24 hours at 62°C.

After the second heating and stirring, the reaction mixture was purified by reprecipitating purification with water and dried under reduced pressure to obtain a yellow solid. The obtained yellow solid was dissolved in chloroform and the chloroform phase was recovered using a separatory funnel. The recovered chloroform phase was concentrated with an evaporator and purified by reprecipitating purification in heptane. The precipitate was collected by filtration and dried under reduced pressure to obtain 7.591 g of temperature-responsive polymer 2, poly(BA-IPAAm). The composition of the obtained temperature-responsive polymer 2 was BA:IPAAm = 32:68 (mol%), the lower critical solution temperature was 32°C, the Mn was $10.8 \times 10^4$, and the Mw/Mn ratio was 1.35.

Reference Example 3: Synthesis method for polymer compound 1 (St/CSt)

After adding 1.156 g (8 mmol) of p-carboxystyrene (CSt, pKa = 4.20) as constituent component (A) and 1.271 g (12 mmol) of styrene (St, HLB value = 0) as constituent component (B) to a 100 mL 2-necked flask, 3.3 mg (20 μmol) of azobisisobutyronitrile and 20 mL of *tert*-butyl alcohol were further added, and nitrogen gas exchange was performed, after which the mixture was heated and stirred for 24 hours at 64°C. Purification was carried out by the same method as Example 1 to obtain 0.92 g of polymer compound 1, poly(CSt/St). The composition of the obtained polymer compound 1 was CSt:St = 33:67 (mol%), the number-average molecular weight Mn was $10.6 \times 10^4$, and the molecular weight distribution Mw/Mn was 1.84.

Reference Example 4: Preparation of surface treatment agent 1

After placing 1.00 g of temperature-responsive polymer 1, 0.01 g of polymer compound 1 and 48.99 g of 1-methoxy-2-propanol in a glass vessel, the mixture was allowed to stand overnight for dissolution. It was then filtered with a 0.22 μm filter (hydrophilic filter, product of Millipore) to obtain surface treatment agent 1.

Reference Example 5: Preparation of surface treatment agent 2

After placing 1.00 g of temperature-responsive polymer 2, 0.01 g of polymer compound 1 and 48.99 g of 1-methoxy-2-propanol in a glass vessel, the mixture was allowed to stand overnight for dissolution. It was then filtered with a 0.22 μm filter (hydrophilic filter, product of Millipore) to obtain surface treatment agent 2.

Reference Example 6: Preparation of surface treatment agent 3

After placing 1.00 g of temperature-responsive polymer 1, 0.05 g of polymer compound 1 and 48.95 g of 1-methoxy-2-propanol in a glass vessel, the mixture was allowed to stand overnight for dissolution. It was then filtered with a 0.22 μm filter (hydrophilic filter, product of Millipore) to obtain surface treatment agent 3.

Reference Example 7: Preparation of temperature-responsive microcarriers 1

A 5 g portion of untreated microcarriers (Product No.: 4625 by Corning, particle diameter: 125 to 212 μm) and 10 g of surface treatment agent 1 were added to a 25 mL volumetric flask and the mixture was allowed to stand for 1 hour. The pressure was then reduced with an evaporator to distill off the solvent and obtain temperature-responsive microcarriers 1.

Reference Example 8: Preparation of temperature-responsive microcarriers 2

A 5 g portion of untreated microcarriers (Product No.: 4625 by Corning, particle diameter: 125 to 212 μm) and 10 g of surface treatment agent 2 were added to a 25 mL volumetric flask and the mixture was allowed to stand for 1 hour. The pressure was then reduced with an evaporator to distill off the solvent and obtain temperature-responsive microcarriers 2.

Reference Example 9: Preparation of temperature-responsive microcarriers 3

A 5 g portion of AmberChrom$^R$ 1 × 8 chloride foam, 100-200 mesh (Product No. 217425, product of Sigma-Aldrich, particle diameter: 54 to 154 $\mu$m) and 10 g of surface treatment agent 3 were added to a 25 mL volumetric flask and the mixture was allowed to stand for 1 hour. The pressure was then reduced with an evaporator to distill off the solvent and obtain temperature-responsive microcarriers 3.

Example 1

After adding 0.6 g of temperature-responsive microcarriers 1 to a 30 mL single use bioreactor (Product No.: BWV-S03A, product of Able Corp.), and seeding 8.64 × 10$^5$ bone marrow-derived human mesenchymal stem cells (Product No.: PT-2501, Lot Number: 0000603525, product of Lonza, Japan), they were treated 10 times (stationing for 59 minutes → stirring for 1 minute at 55 rpm) at 37°C with a $CO_2$ concentration of 5%, and suspension culture was carried out for 4 days at 55 rpm. The culture solution used was 30 mL of mesenchymal stem cell growth medium 2 (Product No.: C-28009, by PromoCell).

After 4 days of culturing, 24 mL of the culture solution was extracted and 24 mL of culture solution cooled to 4°C was added, and then the mixture was allowed to stand for 10 minutes at room temperature (23°C). The culture solution was then stirred at 200 rpm (stirring Reynolds number: 1330) for 5 min to detach the cells from the microcarriers. After stirring, the microcarriers were removed from the culture solution using a 100 $\mu$m mesh cell strainer, to obtain a cell suspension. The collected cell suspension was centrifuged under conditions of 160 rcf, 25°C, 5 min, the supernatant was removed, and PBS(-) was added to produce a suspension. The obtained cell suspension was mixed with a 0.4 w/v% Trypan blue solution at 1:1, 10 $\mu$L was added to a cell counting slide (trade name: Countess Cell Counting Chamber Slide by Thermo Fisher Scientific), and an automatic cell counter (trade name: Countess II by Thermo Fisher Scientific) was used for measurement of the cell count A and viable cell rate of the cell suspension. As a result, the cell count A was 1.12 × 10$^7$ cells and the viable cell rate was 95%. The microcarriers removed with the cell strainer were subjected to enzyme treatment using a trypsin-EDTA solution, and the cell count B of cells that were not detached by the cooling and stirring procedure was measured in the same manner. The values were used to calculate the cell recovery rate by formula (2).

$$A/(A + B) \times 100 \ (\%) \qquad \text{Formula (2)}$$

As a result, the cell count B was 9.74 × 10$^5$ cells and the cell recovery rate was 92%.

Example 2

The same method as Example 1 was carried out, except for using temperature-responsive microcarriers 2. As a result, the cell count A was 6.12 × 10$^6$ cells, the viable cell rate was 92%, the cell count B was 6.80 × 10$^5$ cells and the cell recovery rate was 90%.

Example 3

The method was carried out in the same manner as Example 1, except that after culturing for 4 days, 24 mL of culture solution was extracted, 24 mL of culture solution cooled to 4°C was added, and after standing for 10 minutes at room temperature (23°C), the culture solution was stirred under conditions of 15 rpm (stirring Reynolds number: 100) for 5 min to detach the cells from the microcarriers. As a result, the cell count A was 7.84 × 10$^6$ cells, the viable cell rate was 98%, the cell count B was 3.05 × 10$^6$ cells and the cell recovery rate was 72%.

Example 4

The method was carried out in the same manner as Example 1, except that after culturing for 4 days, 24 mL of culture solution was extracted, 24 mL of culture solution cooled to 4°C was added, and after standing for 10 minutes at room temperature (23°C), the culture solution was stirred under conditions of 270 rpm (stirring Reynolds number: 1800) for 5 min to detach the cells from the microcarriers. As a result, the cell count A was 1.25 × 10$^7$ cells, the viable cell rate was 89%, the cell count B was 1.55 × 10$^6$ cells and the cell recovery rate was 98%.

Example 5

The method was carried out in the same manner as Example 1, except that after adding 0.4 g of temperature-responsive microcarriers 3 to a 30 mL single use bioreactor and seeding $8.64 \times 10^5$ bone marrow-derived human mesenchymal stem cells, they were treated 8 times (stationing for 175 minutes → stirring for 5 minutes at 80 rpm) at 37°C with a $CO_2$ concentration of 5%, and suspension culture was carried out for 4 days at 80 rpm. As a result, the cell count A was $7.75 \times 10^6$ cells, the viable cell rate was 96%, the cell count B was $8.60 \times 10^5$ cells and the cell recovery rate was 90%.

Comparative Example 1

The method was carried out in the same manner as Example 1, except that culture solution cooled to 4°C was added, and after standing for 10 minutes at room temperature (23°C), the culture solution was stirred under conditions of 5 rpm (stirring Reynolds number: 33) for 5 min to detach the cells from the microcarriers. As a result, the cell count A was $2.39 \times 10^6$ cells, the viable cell rate was 88%, the cell count B was $8.47 \times 10^6$ cells and the cell recovery rate was 22%, thus showing a drastically lower cell recovery rate.

Comparative Example 2

The method was carried out in the same manner as Example 1, except that culture solution cooled to 4°C was added, and after standing for 10 minutes at room temperature (23°C), the culture solution was stirred under conditions of 350 rpm (stirring Reynolds number: 2324) for 5 min to detach the cells from the microcarriers. As a result the cell count A was $1.29 \times 10^7$ cells, the viable cell rate was 68%, the cell count B was $8.23 \times 10^5$ cells and the cell recovery rate was 94%, thus showing a drastically lower viable cell rate.

Comparative Example 3

The same method as Example 1 was carried out, except for using untreated microcarriers (Product No.: 4625 by Corning, particle diameter: 125 to 212 $\mu$m). As a result, the cell count A was $4.98 \times 10^5$ cells, the viable cell rate was 92%, the cell count B was $9.47 \times 10^6$ cells and the cell recovery rate was 5%.

Comparative Example 4

The same method was carried out as in Example 5, except for using untreated microcarriers 2 (AmberChrom[R], 1 $\times$ 8 chloride foam, 100-200 mesh (Product No. 217425, product of Sigma-Aldrich, particle diameter: 54 to 154 $\mu$m)). As a result, the cell count A was $2.75 \times 10^5$ cells, the viable cell rate was 85%, the cell count B was $9.68 \times 10^6$ cells and the cell recovery rate was 3%.

[Table 1]

Table 1

| | Microcarrier | Stirring conditions during cell detachment | | Viable cell rate | Cell recovery rate |
|---|---|---|---|---|---|
| | | Stirring speed | Stirring Reynolds number | | |
| Example 1 | Temperature-responsive microcarriers 1 | 200 rpm | 1330 | 95% | 92% |
| Example 2 | Temperature-responsive microcarriers 2 | 200 rpm | 1330 | 92% | 90% |
| Example 3 | Temperature-responsive microcarriers 1 | 15 rpm | 100 | 98% | 72% |
| Example 4 | Temperature-responsive microcarriers 1 | 270 rpm | 1800 | 89% | 98% |
| Example 5 | Temperature-responsive microcarriers 3 | 200 rpm | 1330 | 96% | 90% |
| Comp. Example 1 | Temperature-responsive microcarriers 1 | 5 rpm | 33 | 88% | 22% |
| Comp. Example 2 | Temperature-responsive microcarriers 1 | 350 rpm | 2324 | 68% | 94% |
| Comp. Example 3 | Untreated microcarriers | 200 rpm | 1330 | 92% | 5% |
| Comp. Example 4 | Untreated microcarriers 2 | 200 rpm | 1330 | 85% | 3% |

<Temperature-responsive cell culturing beads and culturing method>

[Analysis of monomer addition rate and temperature-responsive polymer composition]

This was determined by 1H-NMR measurement using Fourier transform nuclear magnetic resonance (NMR). The NMR apparatus used was a JNM-ECZ400S/L1 (JEOL Corp.), and measurement was performed with 10 mg of the temperature-responsive polymer dissolved in 0.75 mL of heavy chloroform.

[Temperature-responsive polymer molecular weight and molecular weight distribution analysis]

The weight-average molecular weight (Mw), number-average molecular weight (Mn) and molecular weight distribution (Mw/Mn) of the temperature-responsive polymer was measured by GPC. The GPC apparatus used was an HLC-8320GPC (Tosoh Corp.). The columns used were two TSKgel Super AWM-H columns (Tosoh Corp.), with the column temperature set to 40°C, a differential refractometer as the concentration detector, and a 10 mM sodium trifluoroacetate/2,2,2-trifluoroethanol solution as the eluent. Measurement was performed under conditions with a sample concentration of 1 mg/mL, a sample injection rate of 0.1 mL and an eluent flow rate of 0.6 mL/min. The calibration curve for molecular weight calculation was drawn by using poly(methyl methacrylate) (PSS Polymer Standards Service GmbH) of known molecular weight, and performing measurement under the same conditions.

[Measurement of thickness of polymer coat layer]

The temperature-responsive beads were exposed to a ruthenium oxide vapor atmosphere for 2 hours to dye the polymer coat layer. The dyed temperature-responsive beads were embedded in an ordinary temperature-curing epoxy resin and slices were prepared using an ultramicrotome. The slices were observed under a transmission electron microscope (JEM-2100F by JEOL Corp.), and the thickness of the layer was measured by image analysis.

Example 6

[Synthesis of temperature-responsive polymer 4]

After adding 1.952 g (15 mmol) of 2-methoxyethyl acrylate (MEA) to a 500 mL cylindrical flask (inner diameter: 80 mm), and further adding 95.1 mg (300 μmol) of cyanomethyldodecyl trithiocarbonate, 4.8 mg (30 μmol) of azobisisobutyronitrile and 30 mL of *tert*-butyl alcohol, argon gas exchange was performed and reaction was conducted for 24 hours at 62°C. The monomer addition rate of the MEA after the reaction was 96%.

After the first heating and stirring, 11.535 g (90 mmol) of *n*-butyl acrylate (BA) was added to the reaction mixture, 4.8 mg (30 μmol) of azobisisobutyronitrile and 10 mL of *tert*-butyl alcohol were further added, and argon gas exchange was performed, after which the mixture was reacted for 24 hours at 62°C. The monomer addition rate of the BA after the reaction was 95%.

After heating and stirring, 22.066 g (195 mmol) of N-isopropylacrylamide (IPAAm:LCST = 32°C) was added to the reaction mixture, 4.8 mg (30 μmol) of azobisisobutyronitrile and 255 mL of *tert*-butyl alcohol were further added, and argon gas exchange was performed, after which the mixture was reacted for 24 hours at 62°C. The monomer addition rate of the IPAAm after the reaction was 99%.

The total amount of the reaction mixture was added dropwise to a 3 L beaker with addition of 2 L of purified water, and the precipitated yellow viscous substance was collected. The viscous substance was immersed in 2 L of purified water for 12 hours, and then heated to 40°C, and the solid was collected and vacuum dried for 12 hours at 100°C. The solid was dissolved in 300 mL of chloroform, and then 5 g of magnesium sulfate was added and the mixture was stirred for 1 hour at room temperature and filtered, collecting the filtrate. The filtrate was added dropwise into a 3 L beaker with addition of 2 L of heptane, and the precipitated white solid was collected and dried under reduced pressure at 100°C for 12 hours to obtain 17.8 g of temperature-responsive polymer 4.

The Mn, the Mw/Mn ratio, the compositional ratio of the temperature-responsive polymer 4 was MEA/BA/IPAAm = 5/30/65 mol%.

[Preparation of surface treatment agent 4]

After placing 0.1 g of temperature-responsive polymer 4 and 49.9 g of 1-methoxy-2-propanol in a glass vessel, the mixture was allowed to stand overnight for dissolution. It was then filtered with a 0.22 μm filter (hydrophilic filter, product of Millipore) to obtain surface treatment agent 4 with a polymer concentration of 0.2 wt%.

[Preparation of temperature-responsive beads 1]

After adding 5 g of calcium phosphate-modified non-porous polystyrene particles and 10 g of surface treatment agent 4 in a 25 mL volumetric flask, the mixture was allowed to stand for 2 hours. The pressure was then reduced with an evaporator to distill off the solvent and obtain temperature-responsive beads 1. The thickness of the coat layer of the temperature-responsive polymer was approximately 58 nm.

[Culturing evaluation]

After adding 60 mg of the PBS(-)-washed temperature-responsive beads 1 into a Costar$^R$ Ultra-low adhesion surface 6-well plate (Product No.: 3471 by Corning), human bone marrow-derived mesenchymal stem cells (Product No. PT-2501, Lot No. 21TL046615, product of Lonza, KK.) were seeded at $1.0 \times 10^5$ cells/well and cultured at 37°C at a 5% $CO_2$ concentration. The culture medium used was 5 mL of mesenchymal stem cell growth medium 2 (Product No.: C-28009, by PromoCell). After culturing for 4 days, 4 mL of culture medium was extracted, 4 mL of medium cooled to 4°C was added, and the mixture was allowed to stand at room temperature for 30 minutes, after which the cell suspension was passed through a cell strainer with a pore size of 100 $\mu$m to recover the cells. The strainer was washed twice with 4 mL of PBS(-), and after centrifugal separation under conditions of 200 g for 5 min together with the cell suspension and removal of the supernatant, it was suspended in culture medium and the number of cells recovered by cooling treatment was counted. The cells that had not been detached by cooling treatment (residual cells) were collected by trypsin enzyme treatment and their number was counted as the residual cell count. The cell proliferation rate was calculated from the total of the number of cells recovered by cooling treatment and the residual cell count, and the cell recovery rate was calculated from the total of the number of cells recovered by cooling treatment and the residual cell count, and the number of cells recovered by cooling treatment. The cell proliferation rate was 590%, and the cell recovery rate by cooling treatment was 86%.

Example 7

[Preparation of temperature-responsive beads 2]

After adding 5 g of quaternary ammonium chloride-modified porous polystyrene particles (product name: AMBERLITE IRA900J Cl by Organo Corp., specific gravity: 1.06) and 10 g of surface treatment agent 4 to a 25 mL volumetric flask, the mixture was allowed to stand for 2 hours. The pressure was then reduced with an evaporator to distill off the solvent and obtain temperature-responsive beads 2. The thickness of the coat layer of the temperature-responsive polymer was 62 nm.

[Culturing evaluation]

This was carried out in the same manner as Example 6, except for using 200 mg of temperature-responsive beads 2. The cell proliferation rate was 710%, and the cell recovery rate was 89%.

Example 8

[Preparation of temperature-responsive beads 3]

After adding 5 g of tertiary amine-modified porous polystyrene particles (product name: AMBERLYST A21 by Organo Corp., specific gravity: 1.07) and 10 g of surface treatment agent 4 to a 25 mL volumetric flask, the mixture was allowed to stand for 2 hours. The pressure was then reduced with an evaporator to distill off the solvent and obtain temperature-responsive beads 3. The thickness of the coat layer of the temperature-responsive polymer was 66 nm.

[Culturing evaluation]

This was carried out in the same manner as Example 6, except for using 90 mg of temperature-responsive beads 3. The cell proliferation rate was 430%, and the cell recovery rate was 91%.

Example 9

[Preparation of surface treatment agent 5]

After placing 0.025 g of temperature-responsive polymer 4 and 49.975 g of 1-methoxy-2-propanol in a glass vessel, the mixture was allowed to stand overnight for dissolution. It was then filtered with a 0.22 $\mu$m filter (hydrophilic filter, product of Millipore) to prepare surface treatment agent 5 with a polymer concentration of 0.05 wt%.

[Preparation of temperature-responsive beads 4]

After adding 5 g of quaternary ammonium chloride-modified porous polystyrene particles (product name: AMBERLITE IRA900J Cl by Organo Corp., specific gravity: 1.06) and 10 g of surface treatment agent 5 to a 25 mL volumetric flask, the mixture was allowed to stand for 2 hours. The pressure was then reduced with an evaporator to distill off the solvent and obtain temperature-responsive beads 4. The thickness of the coat layer of the temperature-responsive polymer was 16 nm.

[Culturing evaluation]

This was carried out in the same manner as Example 6, except for using 200 mg of temperature-responsive beads 4. The cell proliferation rate was 750%, and the cell recovery rate was 83%.

Example 10

[Preparation of surface treatment agent 6]

After placing 0.5 g of temperature-responsive polymer 4 and 49.5 g of 1-methoxy-2-propanol in a glass vessel, the mixture was allowed to stand overnight for dissolution. It was then filtered with a 0.22 $\mu$m filter (hydrophilic filter, product of Millipore) to prepare surface treatment agent 6 with a polymer concentration of 1 wt%.

[Preparation of temperature-responsive beads 5]

After adding 5 g of quaternary ammonium chloride-modified porous polystyrene particles (product name: AMBERLITE IRA900J Cl by Organo Corp., specific gravity: 1.06) and 10 g of surface treatment agent 6 to a 25 mL volumetric flask, the mixture was allowed to stand for 2 hours. The pressure was then reduced with an evaporator to distill off the solvent and obtain temperature-responsive beads 5. The thickness of the coat layer of the temperature-responsive polymer was 253 nm.

[Culturing evaluation]

This was carried out in the same manner as Example 6, except for using 200 mg of temperature-responsive beads 5. The cell proliferation rate was 720%, and the cell recovery rate was 90%.

Example 11

[Preparation of surface treatment agent 7]

After placing 1 g of temperature-responsive polymer 4 and 49 g of 1-methoxy-2-propanol in a glass vessel, the mixture was allowed to stand overnight for dissolution. It was then filtered with a 0.22 $\mu$m filter (hydrophilic filter, product of Millipore) to prepare surface treatment agent 7 with a polymer concentration of 2 wt%.

[Preparation of temperature-responsive beads 6]

After adding 5 g of quaternary ammonium chloride-modified porous polystyrene particles (product name: AMBERLITE IRA900J Cl by Organo Corp., specific gravity: 1.06) and 10 g of surface treatment agent 7 to a 25 mL volumetric flask, the mixture was allowed to stand for 2 hours. The pressure was then reduced with an evaporator to distill off the solvent and obtain temperature-responsive beads 6. The thickness of the coat layer of the temperature-responsive polymer was 698 nm.

[Culturing evaluation]

This was carried out in the same manner as Example 6, except for using 200 mg of temperature-responsive beads 6. The cell proliferation rate was 680%, and the cell recovery rate was 92%.

Example 12

[Synthesis of temperature-responsive polymer 5]

After adding 5.165 g (60 mmol) of *n*-butyl acrylate (BA) to a 500 mL cylindrical flask (inner diameter: 80 mm), and further adding 95.1 mg (300 μmol) of cyanomethyldodecyl trithiocarbonate, 4.8 mg (30 μmol) of azobisisobutyronitrile and 30 mL of *tert*-butyl alcohol, argon gas exchange was performed and reaction was conducted for 24 hours at 62°C. The monomer addition rate of the BA after the reaction was 97%.

After heating and stirring, 27.158 g (240 mmol) of N-isopropylacrylamide (IPAAm:LCST = 32°C) was added to the reaction mixture, 4.8 mg (30 μmol) of azobisisobutyronitrile and 255 mL of *tert*-butyl alcohol were further added, and argon gas exchange was performed, after which the mixture was reacted for 24 hours at 62°C. The monomer addition rate after the reaction was 98%.

The total amount of reaction mixture was added dropwise to a 3 L beaker with addition of 2 L of purified water, and the precipitated yellow viscous substance was collected. The viscous substance was immersed in 2 L of purified water for 12 hours, and then heated to 40°C, and the solid was collected and vacuum dried for 12 hours at 100°C. The solid was dissolved in 300 mL of chloroform, and then 5 g of magnesium sulfate was added and the mixture was stirred for 2 hours at room temperature and filtered, collecting the filtrate. The filtrate was added dropwise into a 3 L beaker with addition of 2 L of heptane, and the precipitated white solid was collected and dried under reduced pressure at 100°C for 12 hours to obtain 21.2 g of temperature-responsive polymer 5.

The Mn, the Mw/Mn ratio, the compositional ratio of the temperature-responsive polymer 5 was BA/IPAAm = 25/75 mol%.

[Preparation of surface treatment agent 8]

After placing 0.1 g of temperature-responsive polymer 5 and 49.9 g of 1-methoxy-2-propanol in a glass vessel, the mixture was allowed to stand overnight for dissolution. It was then filtered with a 0.22 μm filter (hydrophilic filter, product of Millipore) to obtain surface treatment agent 8 with a polymer concentration of 0.2 wt%.

[Preparation of temperature-responsive beads 7]

After adding 5 g of quaternary ammonium chloride-modified porous polystyrene particles (product name: AMBERLITE IRA900J Cl by Organo Corp., specific gravity: 1.06) and 10 g of surface treatment agent 8 to a 25 mL volumetric flask, the mixture was allowed to stand for 2 hours. The pressure was then reduced with an evaporator to distill off the solvent and obtain temperature-responsive beads 7. The thickness of the coat layer of the temperature-responsive polymer was 52 nm.

[Culturing evaluation]

This was carried out in the same manner as Example 6, except for using 200 mg of temperature-responsive beads 7. The cell proliferation rate was 720%, and the cell recovery rate was 71%.

Comparative Example 5

[Preparation of temperature-responsive beads 8]

After adding 5 g of polystyrene carrier (specific gravity: 1.05) and 10 g of surface treatment agent 4 to a 25 mL volumetric flask, the mixture was allowed to stand for 2 hours. The pressure was then reduced with an evaporator to distill off the solvent and obtain temperature-responsive beads 8. The thickness of the coat layer of the temperature-responsive polymer was 52 nm.

[Culturing evaluation]

This was carried out in the same manner as Example 6, except for using 60 mg of temperature-responsive beads 8. The cell proliferation rate was 250%, and the cell recovery rate was 80%.

Comparative Example 6

[Preparation of surface treatment agent 9]

After placing 0.05 g of temperature-responsive polymer 1 and 499.95 g of 1-methoxy-2-propanol in a glass vessel, the mixture was allowed to stand overnight for dissolution. It was then filtered with a 0.22 μm filter (hydrophilic filter, product of Millipore) to obtain surface treatment agent 9 with a polymer concentration of 0.01 wt%.

[Preparation of temperature-responsive beads 9]

After adding 5 g of quaternary ammonium chloride-modified porous polystyrene particles (product name: AMBERLITE IRA900J Cl by Organo Corp., specific gravity: 1.06) and 10 g of surface treatment agent 9 to a 25 mL volumetric flask, the mixture was allowed to stand for 2 hours. The pressure was then reduced with an evaporator to distill off the solvent and obtain temperature-responsive beads 9. The thickness of the coat layer of the temperature-responsive polymer was 5 nm.

[Culturing evaluation]

This was carried out in the same manner as Example 6, except for using 200 mg of temperature-responsive beads 9. The cell proliferation rate was 770%, and the cell recovery rate was 4%.

Comparative Example 7

[Preparation of temperature-responsive beads 10]

After adding 5 g of sulfonic acid-modified porous polystyrene particles (product name: AMBERLITE 200CT Na by Organo Corp., specific gravity: 1.04) and 10 g of surface treatment agent 4 to a 25 mL volumetric flask, the mixture was allowed to stand for 2 hours. The pressure was then reduced with an evaporator to distill off the solvent and obtain temperature-responsive beads 10. The thickness of the coat layer of the temperature-responsive polymer was 55 nm.

[Culturing evaluation]

This was carried out in the same manner as Example 6, except for using 120 mg of temperature-responsive beads 10. The cultured cells were not adhering to the beads, and the cell proliferation rate was 90%.

Comparative Example 8

[Preparation of temperature-responsive beads 11]

After adding 5 g of carboxylic acid-modified porous polystyrene particles (product name: AMBERLITE IRC76 by Organo Corp., specific gravity: 1.04) and 10 g of surface treatment agent 4 to a 25 mL volumetric flask, the mixture was allowed to stand for 2 hours. The pressure was then reduced with an evaporator to distill off the solvent and obtain temperature-responsive beads 11. The thickness of the coat layer of the temperature-responsive polymer was 59 nm.

[Culturing evaluation]

This was carried out in the same manner as Example 6, except for using 140 mg of temperature-responsive beads 11. The cultured cells were not adhering to the beads, and the cell proliferation rate was 90%.

Comparative Example 9

[Culturing evaluation]

This was carried out in the same manner as Example 6, except for using 60 mg of calcium phosphate-modified non-porous polystyrene particles as the beads. The cell proliferation rate was 590%, and the cell recovery rate was 3%.

Comparative Example 10

[Culturing evaluation]

This was carried out in the same manner as Example 6, except for using 200 mg of quaternary ammonium chloride-modified porous AMBERLITE IRA900J Cl (product of Organo Corp., specific gravity: 1.06) as the beads. The cell proliferation rate was 730%, and the cell recovery rate was 4%.

Comparative Example 11

[Culturing evaluation]

This was carried out in the same manner as Example 6, except for using 90 mg of tertiary amine-modified porous AMBERLYST A21 (product of Organo Corp., specific gravity: 1.07) as the beads. The cell proliferation rate was 460%, and the cell recovery rate was 2%.

Example 13

[Preparation of temperature-responsive beads 12]

After adding 5 g of quaternary ammonium chloride-modified porous polystyrene particles (product name: AmberChrom 1 × 8, 200-400 mesh by Sigma-Aldrich Japan, KK., specific gravity: 1.09) and 10 g of surface treatment agent 4 to a 25 mL volumetric flask, the mixture was allowed to stand for 2 hours. The pressure was then reduced with an evaporator to distill off the solvent and obtain temperature-responsive beads 12. The thickness of the coat layer of the temperature-responsive polymer was 50 nm.

[Culturing evaluation]

This was carried out in the same manner as Example 6, except for using 20 mg of temperature-responsive beads 12. The cell proliferation rate was 790%, and the cell recovery rate was 88%.

Example 14

[Preparation of temperature-responsive beads 13]

After adding 5 g of quaternary ammonium chloride-modified porous polystyrene particles (product name: AmberChrom 1 × 8, 100-200 mesh by Sigma-Aldrich Japan, KK., specific gravity: 1.09) and 10 g of surface treatment agent 5 to a 25 mL volumetric flask, the mixture was allowed to stand for 2 hours. The pressure was then reduced with an evaporator to distill off the solvent and obtain temperature-responsive beads 13. The thickness of the coat layer of the temperature-responsive polymer was 12 nm.

[Culturing evaluation]

This was carried out in the same manner as Example 6, except for using 35 mg of temperature-responsive beads 13. The cell proliferation rate was 830%, and the cell recovery rate was 80%.

Example 15

[Preparation of temperature-responsive beads 14]

After adding 5 g of quaternary ammonium chloride-modified porous polystyrene particles (product name: AmberChrom 1 × 8, 100-200 mesh by Sigma-Aldrich Japan, KK., specific gravity: 1.09) and 10 g of surface treatment agent 4 to a 25 mL volumetric flask, the mixture was allowed to stand for 2 hours. The pressure was then reduced with an evaporator to distill off the solvent and obtain temperature-responsive beads 14. The thickness of the coat layer of the temperature-responsive polymer was 61 nm.

[Culturing evaluation]

This was carried out in the same manner as Example 6, except for using 35 mg of temperature-responsive beads 14. The cell proliferation rate was 750%, and the cell recovery rate was 86%.

Example 16

[Preparation of temperature-responsive beads 15]

After adding 5 g of quaternary ammonium chloride-modified porous polystyrene particles (product name: AmberChrom 1 × 8, 50-100 mesh by Sigma-Aldrich Japan, KK., specific gravity: 1.09) and 10 g of surface treatment agent 4 to a 25 mL volumetric flask, the mixture was allowed to stand for 2 hours. The pressure was then reduced with an evaporator to distill off the solvent and obtain temperature-responsive beads 15. The thickness of the coat layer of the temperature-responsive polymer was 44 nm.

[Culturing evaluation]

This was carried out in the same manner as Example 6, except for using 100 mg of temperature-responsive beads 15. The cell proliferation rate was 790%, and the cell recovery rate was 85%.

Comparative Example 12

[Preparation of temperature-responsive beads 16]

After adding 5 g of quaternary ammonium chloride-modified porous polystyrene particles (product name: AmberChrom 1 × 8, 100-200 mesh by Sigma-Aldrich Japan, KK., specific gravity: 1.09) and 10 g of surface treatment agent 9 to a 25 mL volumetric flask, the mixture was allowed to stand for 2 hours. The pressure was then reduced with an evaporator to distill off the solvent and obtain temperature-responsive beads 16. The thickness of the coat layer of the temperature-responsive polymer was 5 nm.

[Culturing evaluation]

This was carried out in the same manner as Example 6, except for using 35 mg of temperature-responsive beads 16. The cell proliferation rate was 750%, and the cell recovery rate was 6%.

[Table 2]

Table 2

| | | Polymer coat layer | | Carrier | | | Culturing evaluation results | |
|---|---|---|---|---|---|---|---|---|
| | | Temperature-responsive polymer | Thickness of layer | Carrier | Carrier surface constituent component | Particl e diameter | Cell proliferation rate | Cell recover y rate |
| Example 6 | | p(MEA/BA/IPAAm) | 58 nm | Calcium phosphate surface polystyrene | Calcium phosphate | 200 $\mu$m | 590% (G) | 86% (G) |
| Example 7 | | p(MEA/BA/IPAAm) | 62 nm | Quaternary ammonium salt surface poly-styrene | $N^+(CH_3)_3C\,l$ | 520 $\mu$m | 710% (G) | 89% (G) |
| Example 8 | | p(MEA/BA/IPAAm) | 66 nm | Tertiary amine surface poly-styrene | $N(CH_3)_2$ | 250 $\mu$m | 430% (G) | 91% (G) |
| Example 9 | | p(MEA/BA/IPAAm) | 16 nm | Quaternary ammonium salt surface poly-styrene | $N^+(CH_3)_3C\,l$ | 520 $\mu$m | 750% (G) | 83% (G) |

(continued)

| | Polymer coat layer | | Carrier | | | Culturing evaluation results | |
|---|---|---|---|---|---|---|---|
| | Temperature-responsive polymer | Thickness of layer | Carrier | Carrier surface constituent component | Particl e diameter | Cell proliferation rate | Cell recover y rate |
| Example 10 | p(MEA/BA/IPAAm) | 253 nm | Quaternary ammonium salt surface poly-styrene | $N^+(CH_3)_3C\,l$ | 520 $\mu$m | 720% (G) | 90% (G) |
| Example 11 | p(MEA/BA/IPAAm) | 698 nm | Quaternary ammonium salt surface poly-styrene | $N^+(CH_3)_3C\,l$ | 520 $\mu$m | 680% (G) | 92% (G) |
| Example 12 | p(BA/IPAAm) | 52 nm | Quaternary ammonium salt surface poly-styrene | $N^+(CH_3)_3C\,l$ | 520 $\mu$m | 720% (G) | 71% (G) |
| Comp. Example 5 | p(MEA/BA/IPAAm) | 52 nm | Polystyrene | - | 200 $\mu$m | 250% (P) | 80% (G) |
| Comp. Example 6 | p(MEA/BA/IPAAm) | 5 nm | Quaternary ammonium salt surface poly-styrene | $N^+(CH_3)_3C\,l$ | 520 $\mu$m | 770% (G) | 4% (P) |
| Comp. Example 7 | p(MEA/BA/IPAAm) | 55 nm | Sulfonic acid salt surface polystyrene | $SO_3Na$ | 360 $\mu$m | 90% (P) | - |
| Comp. Example 8 | p(MEA/BA/IPAAm) | 59 nm | Carboxylic acid salt surface polystyrene | COONa | 400 $\mu$m | 90% (P) | - |
| Comp. Example 9 | - | - | Calcium phosphate surface polystyrene | Calcium phosphate | 200 $\mu$m | 590% (G) | 3% (P) |
| Comp. Example 10 | - | - | Quaternary ammonium salt surface poly-styrene | $N^+(CH_3)_3C\,l$ | 520 $\mu$m | 730% (G) | 4% (P) |
| Comp. Example 11 | - | - | Tertiary amine surface poly-styrene | $N(CH_3)_2$ | 250 $\mu$m | 460% (G) | 2% (P) |

[Table 3]

Table 3

| | Polymer coat layer | | Carrier | | | Culturing evaluation results | |
|---|---|---|---|---|---|---|---|
| | Temperature-responsive polymer | Thickness of layer | Carrier | Carrier surface constituent component | Particle diameter | Cell proliferation rate | Cell recovery rate |
| Example 13 | p(MEA/BA/IPAAm) | 50 nm | Quaternary ammonium salt surface polystyrene | $N^+(CH_3)_3Cl$ | 60 μm | 790% (G) | 88% (G) |
| Example 14 | p(MEA/BA/IPAAm) | 12 nm | Quaternary ammonium salt surface polystyrene | $N^+(CH_3)_3Cl$ | 95 μm | 830% (G) | 80% (G) |
| Example 15 | p(MEA/BA/IPAAm) | 61 nm | Quaternary ammonium salt surface polystyrene | $N^+(CH_3)_3Cl$ | 95 μm | 750% (G) | 86% (G) |
| Example 16 | p(MEA/BA/IPAAm) | 44 nm | Quaternary ammonium salt surface polystyrene | $N^+(CH_3)_3Cl$ | 290 μm | 790% (G) | 85% (G) |
| Comp. Example 12 | p(MEA/BA/IPAAm) | 5 nm | Quaternary ammonium salt surface polystyrene | $N^+(CH_3)_3Cl$ | 95 μm | 750% (G) | 6% (P) |

Cell proliferation rate = (cell count recovered by cooling treatment + residual cell count)/initial seeded count $\times$ 100

Cell recovery rate = cell count recovered by cooling treatment/(cell count recovered by cooling treatment + residual cell count) $\times$ 100

**Claims**

1. A method for culturing adherent cells in a culture vessel using microcarriers coated with a polymer that exhibits a lower critical solution temperature, the method comprising the following steps (1) to (4):

   (1) a step of culturing the cells on the surfaces of the microcarriers in a culture solution at a temperature at or above the lower critical solution temperature,
   (2) following step (1), a step of cooling the culture solution to a temperature at or below the lower critical solution temperature,
   (3) following step (2), a step of stirring the culture solution in the culture vessel to detach the cells from the surfaces of the microcarriers, and
   (4) following step (3), a step of recovering the cells detached from the surfaces of the microcarriers.

2. The method according to claim 1, wherein in step (3), stirring is carried out with a stirring Reynolds number in the range of 50 to 2000.

3. The method according to claim 2, wherein step (2) is carried out by exchanging 10 (v/v)% to 90 (v/v)% of the culture solution used in step (1) with culture solution cooled to a temperature at or below the lower critical solution temperature.

**4.** The method according to claim 3, wherein step (4) is carried out by removing the microcarriers using a mesh.

**5.** The method according to claim 4, wherein the particle diameters of the microcarriers are 50 $\mu$m to 1000 $\mu$m.

**6.** The method according to claim 5, wherein the lower critical solution temperature is 0°C to 50°C.

**7.** The method according to claim 6, wherein the adherent cells are stem cells.

**8.** Temperature-responsive beads comprising a carrier and a polymer coat layer made of a temperature-responsive polymer having a lower critical solution temperature of 0°C to 50°C, wherein the carrier surface is positively charged, and the thickness of the layer of the temperature-responsive polymer is 10 nm to 1000 nm.

**9.** Temperature-responsive beads according to claim 8, wherein the positively charged component of the carrier surface includes a tertiary amine, a quaternary ammonium salt or an alkaline earth metal salt.

**10.** The beads according to claim 9, wherein the specific gravity of the carrier is 1.0 to 1.1.

**11.** The temperature-responsive beads according to claim 10, wherein the material of the carrier is polystyrene.

**12.** The temperature-responsive beads according to claim 11, wherein the particle diameter of the carrier is 50 $\mu$m to 1000 $\mu$m.

**13.** A cell culturing method using temperature-responsive beads according to claim 12.

# INTERNATIONAL SEARCH REPORT

| International application No. |
|---|
| **PCT/JP2023/036049** |

## A. CLASSIFICATION OF SUBJECT MATTER

*C12N 5/07*(2010.01)i; *C08J 3/12*(2006.01)i; *C12M 1/00*(2006.01)i; *C12M 3/00*(2006.01)i; *C12N 1/02*(2006.01)i;
*C12N 5/00*(2006.01)i; *C12N 5/071*(2010.01)i; *C12N 5/0735*(2010.01)i; *C12N 5/074*(2010.01)i; *C12N 5/0775*(2010.01)i;
*C12N 5/0789*(2010.01)i; *C12N 5/0797*(2010.01)i
FI: C12N5/07; C12N5/071; C12M3/00 A; C12M1/00 A; C12N5/0775; C12N5/00; C12N5/0735; C12N5/074; C12N5/0789; C12N5/0797; C12N1/02; C08J3/12 Z CER; C08J3/12 CEZ; C12M1/00 C

According to International Patent Classification (IPC) or to both national classification and IPC

## B. FIELDS SEARCHED

Minimum documentation searched (classification system followed by classification symbols)

C12N5/07; C08J3/12; C12M1/00; C12M3/00; C12N1/02; C12N5/00; C12N5/071; C12N5/0735; C12N5/074; C12N5/0775; C12N5/0789; C12N5/0797

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

## C. DOCUMENTS CONSIDERED TO BE RELEVANT

| Category* | Citation of document, with indication, where appropriate, of the relevant passages | Relevant to claim No. |
|---|---|---|
| X | JP 2021-159049 A (DAIKIN INDUSTRIES, LTD.) 11 October 2021 (2021-10-11) | 1-2 |
| | claims, paragraphs [0056], [0058]-[0061], [0130], example 1 | |
| Y | | 3-13 |
| X | WO 2012/029877 A1 (CELLSEED INC.) 08 March 2012 (2012-03-08) | 1-2 |
| | claims 1, 4, 5, paragraphs [0017], [0021], [0025], [0030], [0031], [0033]-[0037] | |
| Y | | 3-13 |
| Y | JP 2021-114995 A (TOSOH CORP) 10 August 2021 (2021-08-10) | 3-7 |
| | paragraphs [0024], [0025] | |
| Y | JP 2020-110140 A (TOSOH CORP.) 27 July 2020 (2020-07-27) | 3-7 |
| | claim 2, paragraph [0038] | |
| Y | JP 2019-126326 A (TOSOH CORP.) 01 August 2019 (2019-08-01) | 8-13 |
| | paragraph [0034] | |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **01 December 2023** | **19 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/036049**

**C.    DOCUMENTS CONSIDERED TO BE RELEVANT**

| Category* | Citation of document, with indication, where appropriate, of the relevant passages | Relevant to claim No. |
|---|---|---|
| A | JIS使い方シリーズ 新版 プラスチック材料選択のポイント. 第2版. Japanese Standards Association, 2003, ISBN: 4-542-30396-9, pp. 436, 437, non-official translation (JIS How To Series - New Edition: Points for Selecting Plastic Materials. 2nd Edition.) in particular, p. 437, Polystyrene Density | 8-13 |

Form PCT/ISA/210 (second sheet) (January 2015)

## INTERNATIONAL SEARCH REPORT
### Information on patent family members

International application No.

**PCT/JP2023/036049**

| Patent document cited in search report | | | Publication date (day/month/year) | Patent family member(s) | Publication date (day/month/year) |
|---|---|---|---|---|---|
| JP | 2021-159049 | A | 11 October 2021 | (Family: none) | |
| WO | 2012/029877 | A1 | 08 March 2012 | JP 2017-12172 A claims 1, 4, 5, paragraphs [0017], [0021], [0025], [0030], [0031], [0033]-[0037] | |
| JP | 2021-114995 | A | 10 August 2021 | (Family: none) | |
| JP | 2020-110140 | A | 27 July 2020 | (Family: none) | |
| JP | 2019-126326 | A | 01 August 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6313822 B
- JP 6954047 B
- JP 2021106543 A
- JP 5846584 B
- JP 7127330 B